# EUROPEAN PATENT APPLICATION

(11) **EP 4 813 298 A1**
(43) Date of publication of application: **30.09.2026**
(21) Application number: 25166113.8
(22) Date of filing: 25.03.2025
(51) Int. Cl.: A61B 5/384

(54) **DATA SIGNAL FOR MULTICHANNEL BIOMEDICAL SIGNAL**

(71) Applicant: Koninklijke Philips N.V., 5656 AE Eindhoven (NL)
(72) Inventor: OOMEN, Arnoldus Werner Johannes, 5656 AG Eindhoven (NL); TSONEVA, Tsvetomira Kirova, 5656 AG Eindhoven (NL); JELFS, Sam Martin, 5656 AG Eindhoven (NL)
(74) Representative: Philips Intellectual Property & Standards

(57) **Abstract**

An apparatus generates a biomedical data signal representing a multichannel biomedical signal comprising biomedical channel signals for measured differential voltages between two measurement electrodes. A determination circuit (103) derives a reference signal from the biomedical channel signals, e.g. as an average signal of the channel signals. A compensation circuit (105) generates compensated biomedical channel signals by compensating the biomedical channels signals for the first reference signal, e.g. by subtracting the reference signal. A data encoder (107) generates encoded compressed data for the compensated biomedical channel signals and a data signal generator (109) generates the biomedical data signal to comprise the compressed encoded data. An apparatus may receive the biomedical data signal and perform the reverse operation of first determining compensated channel signals and then compensating these by the reference signal to generate the biomedical data signal.

## Description

### FIELD OF THE INVENTION

The invention relates to generating and/or processing a data signal for a multichannel biomedical signal, and in particular, but not exclusively, for an electrocardiography (ECG), electroencephalography (EEG), or electromyography (EMG) signal.

### BACKGROUND OF THE INVENTION

The capturing, storing, distributing, and processing of biomedical data has become increasingly important in recent decades as recording such signals has progressed from being performed almost exclusively for short term measurements in dedicated clinical environments, such as hospitals, to becoming increasingly prevalent in the personal space with biomedical measurements often being made continuously over very long periods. For example, the cardiology field has evolved from mainly considering short heart rate waveforms collected using chest-worn outpatient devices that are routinely worn for multiple days, and which collect continuous data for long durations. Such measurements typically include simultaneous measurements at different positions and result in a multichannel biomedical signal with a high level of information content.

Consequently, the requirements for data storage, distribution, and processing have grown exponentially. Accordingly, there is a desire to effectively represent captured biomedical waveform signals to facilitate and reduce the requirements for storing, distributing, and/or processing the signals. However, whereas various proprietary approaches have been proposed for encoding or compressing biomedical waveform signals used by various manufacturers, current approaches tend to be suboptimal and tend to not provide ideal performance. Further, current approaches tend to be complex and/or be targeted on specific biomedical waveform signals resulting in specific requirements and limited application.

Hence, an improved approach for representing multichannel biomedical signals would be advantageous. In particular, an approach allowing increased flexibility, improved performance, increased quality, reduced complexity, reduced data rate, increased accuracy/reduced distortion of encoding/decoding/compressing/decompressing operations, reduced storage requirements, reduced bandwidth/data rate for biomedical waveform signals, reduced computational load, facilitated implementation, and/or improved performance would be advantageous.

### SUMMARY OF THE INVENTION

Accordingly, the invention seeks to preferably mitigate, alleviate or eliminate one or more of the above-mentioned disadvantages singly or in any combination.

According to an aspect of the invention, there is provided an apparatus for generating a biomedical data signal, the apparatus comprising: a receiver arranged to receive a multichannel biomedical signal comprising a plurality of biomedical channel signals, each biomedical channel signal representing a measured differential voltage between two measurement electrodes; a determination circuit arranged to derive a first reference signal from the plurality of biomedical channel signals; a compensation circuit arranged to generate compensated biomedical channel signals by compensating at least some of the plurality of biomedical channels signals for the first reference signal; a data encoder arranged to generate encoded compressed data for the compensated biomedical channel signals; and a data signal generator arranged to generate the biomedical data signal to comprise the compressed encoded data.

The invention may allow advantageous generation of a biomedical data signal representing a multichannel biomedical signal. It may in many embodiments allow an improved data rate to quality trade-off, and may in many embodiments provide advantageous compression/encoding of the multichannel biomedical signal. The invention may allow a reduced bandwidth/data rate for compressed encoded data representing a multichannel biomedical signal. In many embodiments, the storage requirements and/or required communication bandwidth may be reduced substantially. The approach may allow efficient and practical implementation and/or may reduce complexity and processing/computational requirements.

The biomedical waveform signal may specifically be an electrocardiography (ECG), electroencephalography (EEG), or electromyography (EMG) signal but any other bioelectrical signals, such as, but not limited to, Electrocorticogram (ECoG), Electrooculogram (EOG), Electroretinogram (ERG), Fetal electrocardiography (fECG), Electrohysterogram (EHG), Electrodermal Activity (EDA), or Electrogastrogram (EGG) could also apply.

Generating the encoded compressed data may include applying a data compression algorithm to the compensated biomedical channel signals. The data encoder is arranged to apply a data compression algorithm to the compensated biomedical channel signals.

In some embodiments, a plurality, and possibly all, biomedical channel signals represent a measured differential voltage between a common reference electrode and a measurement electrode for the biomedical channel signal. In some embodiments, a first biomedical channel signal represents a measured differential voltage between a first pair of electrodes and a second biomedical channel signal represents a measured differential voltage between a second pair of electrodes, the first pair of electrodes not including any electrode of the second pair of electrodes.

According to an optional feature of the invention, data signal generator is arranged to include a compensation indication in the biomedical data signal, the compensation indication being indictive of a property of the compensation of the at least some of the plurality of biomedical channels signals.

This may provide particularly advantageous operation and/or performance in many embodiments and scenarios.

The compensation indication may be included as metadata. The compensation indication may be indicative of whether the compensation has been performed or not. The compensation indication may be indicative of how the compensation has been performed and/or of how the reference signal has been determined. In many embodiments, the compensation indication may provide an indication of a property of the derivation of the reference signal. In some embodiments, the compensation indication may be indicative of a function used to determine the reference signal.

According to an optional feature of the invention, the data encoder is arranged to generate encoded reference signal data for the first reference signal; and the data signal generator is arranged to generate the biomedical data signal to comprise the encoded reference signal data.

This may provide particularly advantageous operation and/or performance in many embodiments and scenarios.

According to an optional feature of the invention, the determination circuit is arranged to select a biomedical channel signal of the plurality of biomedical channel signals as the first reference signal.

This may provide particularly advantageous operation and/or performance in many embodiments and scenarios. It may typically provide reduced complexity and/or a reduced data rate.

According to an optional feature of the invention, the determination circuit is arranged to generate the first reference signal as a combination of at least some of the biomedical channel signals. This may provide particularly advantageous operation and/or performance in many embodiments and scenarios. It may often provide an improved first reference signal allowing a reduced encoded data rate for the biomedical channel signals. The combination may be a linear combination, and specifically may be an averaging/summation.

According to an optional feature of the invention, the determination circuit is arranged to generate the first reference signal as a signal that reduces a difference measure, the difference measure being indicative of a difference between the first reference signal and a set of signals from the plurality of biomedical channel signals.

This may provide particularly advantageous operation and/or performance in many embodiments and scenarios.

In some embodiments, the determination circuit may be arranged to generate the first reference signal as a signal for which a difference measure indicative of a difference between the first reference signal and a set of biomedical channel signals is minimized/optimized.

In some embodiments, the determination circuit may be arranged to generate the first reference signal as a signal for which a statistical measure indicative of a difference between the first reference signal and a set of biomedical channel signals is minimized/optimized.

In some embodiments, the determination circuit is arranged to determine the first reference signal as a biomedical channel signal having a highest combined correlation with the other biomedical channel signals.

According to an optional feature of the invention, the compensation circuit is arranged to determine the at least some of the plurality of biomedical channels signals as a first subset of biomedical channel signals.

This may provide particularly advantageous operation and/or performance in many embodiments and scenarios.

In many embodiments, the determination circuit is arranged to determine the first reference signal from only biomedical channel signals comprised in the first subset of the biomedical channel signals.

According to an optional feature of the invention, the data signal generator is arranged to include an indication of the first subset in the biomedical data signal.

This may provide particularly advantageous operation and/or performance in many embodiments and scenarios.

According to an optional feature of the invention, the determination circuit is arranged to derive a second reference signal from the plurality of biomedical channel signals; and the compensation circuit is arranged to generate further compensated biomedical channel signals by compensating a second set of biomedical channel signals by the second reference signal; the data encoder is arranged to generate further encoded compressed data for the further compensated biomedical channel signals; and the data signal generator is arranged to generate the biomedical data signal to comprise the further compressed encoded data.

This may provide particularly advantageous operation and/or performance in many embodiments and scenarios.

In many embodiments, the determination circuit is arranged to determine the second reference signal from only biomedical channel signals comprised in the second subset of the biomedical channel signals.

In some embodiments, the compensation circuit is arranged to generate at least one compensated biomedical channel signal by compensating a biomedical channel signal by a combination of the first reference signal and the second reference signal; the data encoder is arranged to generate encoded compressed data for the compensated biomedical channel signal; and the data signal generator is arranged to generate the biomedical data signal to comprise this compressed encoded data.

According to an optional feature of the invention, the data signal generator is arranged to include an indication of the second subset in the biomedical data signal.

This may provide particularly advantageous operation and/or performance in many embodiments and scenarios.

According to an optional feature of the invention, the data encoder is arranged to generate further encoded compressed data by encoding the second reference signal relative to the first reference signal.

This may provide particularly advantageous operation and/or performance in many embodiments and scenarios.

According to an optional feature of the invention, the data signal generator is arranged to include an indication indicative of the second reference signal being encoded relative to the first reference signal.

This may provide particularly advantageous operation and/or performance in many embodiments and scenarios.

In some embodiments, the determination circuit is arranged to determine a third reference signal from the plurality of biomedical channel signals; and the compensation circuit is arranged to generate further compensated biomedical channel signals by compensating a third set of biomedical channel signals by the third reference signal; the data encoder is arranged to generate further encoded compressed data for these further compensated biomedical channel signals; and the data signal generator arranged to generate the biomedical data signal to comprise this further compressed encoded data.

The encoder may be arranged to generate further encoded compressed data by encoding the third reference signal relative to a combination of the first reference signal and the second reference signal.

According to an optional feature of the invention, the determination circuit is arranged to determine the first reference signal in dependence on measurement positions for the measurement electrodes.

This may provide particularly advantageous operation and/or performance in many embodiments and scenarios.

According to another aspect of the invention, there is provided an apparatus for generating a multichannel biomedical signal, the apparatus comprising: a receiver arranged to receive a biomedical data signal comprising compressed encoded data for a multichannel biomedical signal comprising a plurality of biomedical channel signals, each biomedical channel signal representing a measured differential voltage between two measurement electrodes and the compressed encoded data representing an encoding of at least some of the biomedical channel signals relative to a reference signal; a determination circuit arranged to determine the reference signal from the biomedical data signal; a decoding circuit arranged to generate intermediate biomedical channel signals by decoding the compressed encoded data; a circuit arranged to generate the biomedical channel signals by (de)compensating at least one of the intermediate biomedical channels by the reference signal; and an output circuit arranged to generate the multichannel biomedical signal to comprise the biomedical channel signals.

According to another aspect of the invention, there is provided a method of generating a biomedical data signal, the method comprising: receiving a multichannel biomedical signal comprising a plurality of biomedical channel signals, each biomedical channel signal representing a measured differential voltage between two measurement electrodes; deriving a first reference signal from the plurality of biomedical channel signals; generating compensated biomedical channel signals by compensating at least some of the plurality of biomedical channels signals for the first reference signal; generating encoded compressed data for the compensated biomedical channel signals; and generating the biomedical data signal to comprise the compressed encoded data.

According to another aspect of the invention, there is provided a method of generating a multichannel biomedical signal, the method comprising: receiving a biomedical data signal comprising compressed encoded data for a multichannel biomedical signal comprising a plurality of biomedical channel signals, each biomedical channel signal representing a measured differential voltage between two measurement electrodes and the compressed encoded data representing an encoding of at least some of the biomedical channel signals relative to a reference signal; determining the reference signal from the biomedical data signal; generating intermediate biomedical channel signals by decoding the compressed encoded data; generating the biomedical channel signals by (de)compensating at least one of the intermediate biomedical channels by the reference signal; and generating the multichannel biomedical signal to comprise the biomedical channel signals.

These and other aspects, features and advantages of the invention will be apparent from and elucidated with reference to the embodiment(s) described hereinafter.

### BRIEF DESCRIPTION OF THE DRAWINGS

Embodiments of the invention will be described, by way of example only, with reference to the drawings, in which
FIG. 1 illustrates some elements of an example of an apparatus for generating a biomedical data signal in accordance with some embodiments of the invention;
FIG. 2 illustrates some elements of an example of an apparatus for generating a multichannel biomedical signal in accordance with some embodiments of the invention;

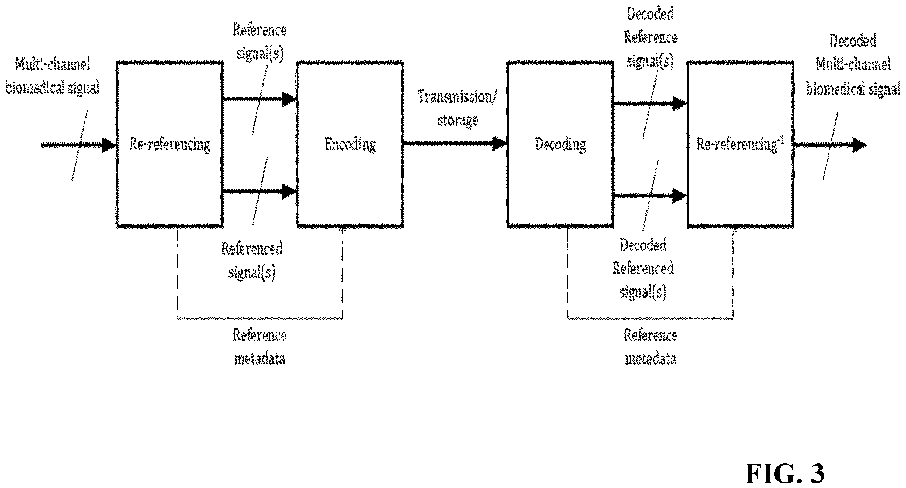

FIG. 3 illustrates an example of some elements of a system for communicating a multichannel biomedical signal in accordance with some embodiments of the invention; and
FIG. 4 illustrates some elements of a possible arrangement of a processor for implementing elements of an apparatus of FIG. 1 or 2 in accordance with some embodiments of the invention.

### DETAILED DESCRIPTION OF THE EMBODIMENTS

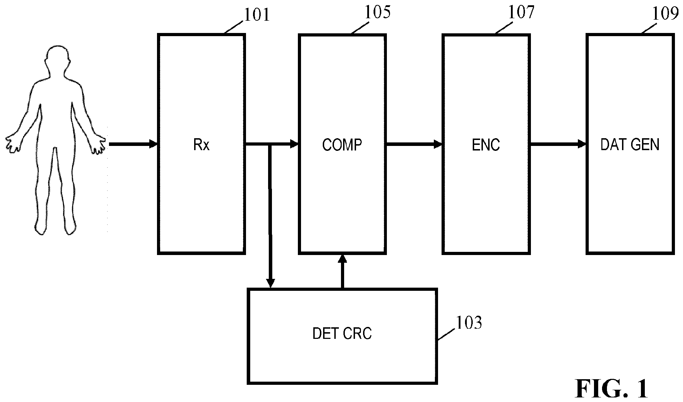

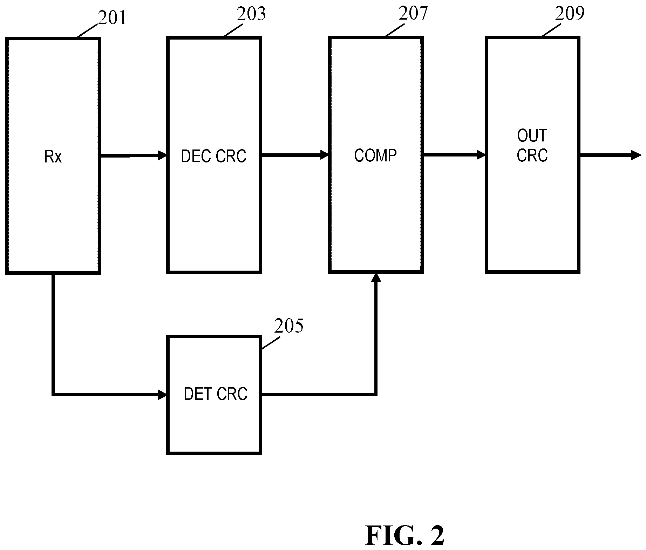

FIG. 1 illustrates an example of an apparatus for generating a biomedical data signal comprising data representing a multichannel biomedical signal. FIG. 2 illustrates an example of an apparatus for generating/recreating a multichannel biomedical signal from a biomedical data signal representing such a multichannel biomedical signal. The apparatus of FIG. 1 will be referred to as the encoder apparatus and the apparatus of FIG. 2, which specifically may receive the biomedical data signal from the encoder apparatus, will be referred to as the decoder apparatus.

The encoder apparatus comprises a receiver 101 which receives a multichannel biomedical signal, which comprises a plurality of biomedical channel signals. Each of the biomedical channel signals represents a measured differential voltage between two measurement electrodes. The measurement electrodes may be positioned in contact with/attached to a patient's/subject's body and each biomedical channel signal may be generated by measuring the voltage difference between two attached electrodes. The number of electrodes and/or channels may depend on the specific application and usage and may range from a few electrodes/channels up to hundreds of electrodes/channels. In some embodiments, each biomedical channel signal may be a differential voltage relative to a common electrode. Thus, the biomedical channel signals may all represent a differential voltage with respect to the same reference electrode. In other embodiments, each biomedical channel signal may be a differential voltage between separate pairs of electrodes and thus a different reference electrode may be used for each biomedical channel signal. In other embodiments, the biomedical channel signals may be divided into groups with the signals of each group being a differential voltage relative to a common reference or electrode for the group, but with this common reference or electrode being different for different groups. For example, reference electrodes may be distributed across the measurement area, and the differential voltage for electrodes may be measured relative to the reference electrode closest to the given electrode, or a linear combination of all electrodes surrounding the electrode/group, etc.

In many embodiments, the multichannel biomedical signal may be an electrocardiography (ECG) signal which specifically may be provided from a sensor arrangement comprising a plurality of electrodes that are positioned on the subject's body to pick up electrical signals relating to the heart operation.

In some embodiments, the multichannel biomedical signal may be an electroencephalography (EEG) signal which specifically may be provided from a sensor comprising a number of electrodes that are positioned on the subject's head to pick up electrical signals relating to brain activity.

In some embodiments, the multichannel biomedical signal may be an electromyography (EMG) signal which specifically may be provided from a sensor comprising a plurality of electrodes that are positioned on the subject's body to pick up electrical signals relating to muscle movement of the person.

It will be appreciated that the multichannel biomedical signal may in different embodiments be any suitable bioelectrical signals.

Each of the biomedical channel signals of the multichannel biomedical signal is specifically a time domain signal with a time domain waveform that reflects the changes in the differential voltage from an electrode relative to a reference electrode.

The encoder apparatus proceeds to process the multichannel biomedical signal to generate a biomedical data signal which includes data that represents/describes the multichannel biomedical signal. The biomedical data signal may be generated to comprise encoded data representing the multichannel biomedical signal (which itself may be represented/received as data values, such as e.g. represented by digital signals representing measured voltages after an A/D conversion.

However, rather than directly encode and represent the received channel signals, the encoder apparatus may perform a re-referencing on the biomedical channel signals such that compensated biomedical channel signals are generated which represent the signals with respect to (at least one) reference signal being derived from the received multichannel biomedical signal. Thus, rather than the biomedical channel signals being differential voltage signals with respect to a second electrode, the biomedical channel signals are re-referenced to be relative to a reference signal generated by the encoder apparatus from the multichannel biomedical signal.

The receiver 101 is accordingly coupled to a determination circuit 103 which is arranged to determine a reference signal from the plurality of biomedical channel signals. As a specific example, the reference signal may be generated as the average signal for all the biomedical channel signals, i.e. for a given time instant, the reference signal may be determined as the average value of all the biomedical channel signal values. It will be appreciated that a number of different approaches may be applied to determine the reference signal depending on the preferences and requirements for the individual embodiment and application as will be described in more detail later.

The receiver 101 and the determination circuit 103 are coupled to a compensation circuit 105 that generates compensated biomedical channel signals by compensating at least some, and often all, of the biomedical channel signals for the reference signal. In many embodiments, the compensation circuit 105 may be arranged to subtract the reference signal from the biomedical channel signals being compensated, and thus the compensated signals may be differential voltage signals relative to a (potentially virtual) reference voltage potential represented by the reference signal. The compensated biomedical channel signals may thus be signals corresponding to the input biomedical channel signals but re-referenced with respect to the generated reference signal.

The compensation circuit 105 is coupled to a data encoder 107 which is arranged to generate encoded compressed data for the compensated biomedical channel signals. The data encoder 107 may be a lossy encoding and may include a data compression to reduce the number of bits required to represent the compensated biomedical channel signals.

The data encoder 107 may be arranged to perform a compression process to generate compressed data from the compensated biomedical channel signals. The compression may be an encoding/compression which results in a representation of the compensated biomedical channel signals which has a reduced data rate/size relative to the compensated biomedical channel signals themselves. The compression may in some cases be a lossy compression and may in some cases be a lossless compression.

The data encoder 107 may first perform a compression operation to generate compressed data which may then be encoded as part of being included in the biomedical data signal. Thus, the data of the biomedical data signal may be encoded compressed data.

The encoding/compression may include operations to remove redundancy by applying lossless coding techniques such as prediction, entropy coding, etc. In many cases, the data encoder 107 may also be arranged to remove irrelevancy from the compensated biomedical channel signals by applying lossy coding techniques such as quantization, noise substitution, etc.

Redundancy removal may preserve the perfect reconstruction of the multichannel biomedical signal. After irrelevancy removal, perfect reconstruction is generally no longer feasible.

The removal of irrelevance is often obtained e.g., by simple thresholding (quantization, e.g., setting coefficients less than a given threshold to zero). For entropy coding various techniques and methods are available, such as Huffman coding or Arithmetic coding.

The data encoder 107 is coupled to a data signal generator 109 which generates the biomedical data signal to comprise the compressed encoded data. The data signal generator 109 may generate a bitstream in accordance with a suitable data format that includes data fields which can carry the compressed encoded data. The data signal generator 109 may generate the biomedical data signal to further include metadata that e.g., may provide information on the compensation/re-referencing performed by the compensation circuit 105. It may further include suitable overhead data required e.g., for communication, synchronization etc.

The biomedical data signal may be provided to the decoder apparatus of FIG. 2. The biomedical data signal may be distributed or communicated in any suitable way including real time communication, storage on storage devices, communication over a network etc.

The decoder apparatus comprises a decoder receiver 201 which receives a biomedical data signal from any internal or external data source. For example, the decoder receiver 201 may extract the biomedical data signal from an internal storage or from a portable storage medium, may receive it via a network interface (e.g., via an Internet connection) etc. The decoder receiver 201 may be arranged to parse/interpret a received bitstream to extract the relevant data.

The decoder apparatus further comprises a decoding circuit 203 arranged to generate intermediate biomedical channel signals by decoding the compressed encoded data of the biomedical data signal. The decoding circuit 203 may perform the complementary operation to the encoding of the encoder apparatus and may thus generate local replicas of the compensated biomedical channel signals. Thus, the intermediate biomedical channel signals may be recreations of the compensated biomedical channel signals.

The decoder apparatus comprises a decoder determination circuit 205 which is arranged to determine the reference signal from the biomedical data signal. In many cases, the reference signal may be explicitly encoded in the biomedical data signal and the decoder determination circuit 205 may simply decode the received encoding data for the reference signal.

The decoding circuit 203 and the decoder determination circuit 205 are coupled to a modification circuit 207 which generates biomedical channel signals by (de)compensating the intermediate biomedical channels by the reference signal. The modification circuit 207 may specifically be arranged to perform the complementary/reverse operation to that performed by the compensation circuit 105 of the encoder apparatus. The modification circuit 207 may accordingly reverse the operation/signal processing/effect of the re-referencing performed at the encoder apparatus. In particular, in many embodiments, the modification circuit 207 is arranged to add the reference signal to each of the decoded intermediate channel signals.

The modification circuit 207 may accordingly modify the received intermediate channel signals to compensate for the re-referencing performed by the encoder apparatus. It generates biomedical channel signals that are local replicas/estimates of the biomedical channel signals of the multichannel biomedical signal input to the encoder apparatus.

The recreated biomedical channel signals are fed to an output circuit 209 which generates the multichannel biomedical signal to comprise the biomedical channel signals. The generated multichannel biomedical signal may be provided in any suitable way.

The approach may provide a highly advantageous coding (and typically compression) of a multichannel biomedical signal. An improved quality/accuracy to data rate trade-off can be achieved in many situations and for many scenarios and applications. Further, the approach can be implemented with low complexity and/or computational resource requirements.

In more detail, e.g., amplifiers for bio-electrical signal measurements, such as electroencephalography (EEG), electrocardiography (ECG), and electromyography (EMG), measure voltages relative to reference and ground electrodes, in order to provide stable and meaningful captures and recordings. The reference electrode serves as the baseline voltage against which the active electrodes' signals are measured and further a ground electrode helps reduce electrical noise and interference by providing a common return path for the amplifier. The choice of reference electrode is often selected as a position/location where no bioelectrical activity of interest is measured, and the ideal position varies depending on the signal type. For EEG, common references include the mastoid, linked ears, or a centrally located electrode (e.g., Cz in the 10-20 system). For ECG, a single limb electrode (e.g., right leg) or Wilson's central terminal (averaged limb electrodes) is often used. For EMG, a nearby electrically neutral site is often preferred. However, despite seeking to optimize positions for reference electrodes, the reference is not always ideal for all electrodes or conditions.

Re-referencing is performed to improve signal quality, reduce common noise, and enhance spatial resolution. One re-referencing method is to use the average reference, where the mean signal across all electrodes is subtracted from each biomedical channel signal to reduce common noise. Another method is to use the linked mastoid reference, which averages signals from two mastoid electrodes to create a more stable reference. The common average reference (CAR) may be used e.g., in high-density EEG and intracranial recordings, helping to minimize spatially widespread noise.

In the approach of FIGs. 1 and 2, the encoding of a multichannel biomedical signal includes a compensation/re-referencing based on a reference signal generated from the biomedical channel signals of the multichannel biomedical signal.

Such re-referencing can help reduce entropy in the biomedical signal measurements by reducing noise and artifacts that contribute to randomness. E.g., by aligning the biomedical channel signals to a more stable baseline, re-referencing can improve data consistency, potentially making it more predictable and compressible.

In the described approach, a compression ratio for a lossless and / or lossy compression of the multichannel biomedical signals may be substantially increased. This is achieved by re-referencing each (or at least some) of the biomedical channels to one or more reference channels. This is specifically done such that the variance between the biomedical channel signals is minimized. This is of particular use for EEG signals, where there is potentially a high number of channels, but may also be useful for other signal types such as e.g., ECG. In some embodiments, a reference channel that all other channels are re-referenced to may be one of the already existing channels, while in other embodiments it may be a new channel that is derived from the existing channels, such as the Common Average Reference. In the latter case the number of channels is increased, but due to the reduced variation between the other channels, greater compression ratios may be achieved. Typically, re-referencing may be applied to the entire multichannel biomedical signal, but in some cases it may only be applied to a subset of biomedical channel signals. In some cases, the compression/encoding may be performed in frames and in such cases the described operation, and specifically the re-referencing, may be applied on a frame-by-frame basis.

In many embodiments, the data signal generator 109 is arranged to include metadata in the biomedical data signal which describes one or more properties of the compensation of the biomedical channel signals. The data signal generator 109 may be arranged to include a compensation indication in the biomedical data signal where the compensation indication is indicative of a property of the compensation performed by the compensation circuit 105.

In some embodiments, the compensation indication may be a single data bit flag, such as e.g., an indication whether the compensation has been performed or not. The compensation indication may in some embodiments be a flag that indicates whether the compressed encoded data represents compensated biomedical channel signals or non-compensated biomedical channel signals. Such an approach may be suitable e.g., for situations where the compensation may be a predetermined operation/compensation. For example, the encoder apparatus may be arranged to derive a reference signal from the biomedical channel signals and to include the resulting reference signal in the biomedical data signal, or e.g., if the reference signal is also a biomedical channel signal, it may include a reference signal simply be indicating that one of the included signals is a reference signal. The decoder apparatus may then be arranged to decode the compressed encoded data to recreate the biomedical channel signals. If the compensation indication indicates that compensation has been applied, it may further proceed to recreate the reference signal and combine this with (typically add to) the biomedical channel signals. If the compensation indication indicates that no compensation has been performed, the decoded biomedical channel signals may be used directly without any compensation.

In other embodiments, a compensation indication may be included which provides substantial information on the compensation that has been performed. For example, the compensation indication may include data describing how the compensation has been performed (e.g., whether a straight subtraction has been performed or whether more complex operations have been performed such as e.g. whether filtering, weighting, etc. has been included in the compensation). In some embodiments, the compensation indication may include data that indicates how the reference signal has been generated, such as e.g., whether it has been selected as one of the biomedical channel signals, is a combination of biomedical channel signals, etc.

The data signal generator 109 may in some embodiments be arranged to include a compensation indication that provides an indication of a function used to derive the reference signal.

In some embodiments, the compensation indication may provide a direct indication of the reference signal, such as for example by indicating that one of the received biomedical channel signals is to be used as a reference signal.

Indeed, in some embodiments, compressed encoded data that represents an encoding of a reference signal may directly be considered a compensation indication e.g., if it is also used as an indication that compensation has been performed. For example, if a dedicated encoded reference signal is included in the biomedical data signal, the decoder apparatus may interpret this as an indication that compensation has been performed and it may accordingly proceed to decode the reference signal and e.g., add this to the received encoded biomedical channel signals.

As an example, the compensation indication may provide an indication of one of the biomedical channel signals (e.g., a channel index indicator). The compensation indication may further e.g., optionally provide information on e.g., a gain factor with which the indicated channel should be scaled. As another example, the compensation indication could indicate multiple biomedical channel signals (e.g., multiple channel indices) along with linear combination weights that should be applied to these in order for a local linear combination to generate the desired reference signal. As another example, the compensation indication may indicate that the reference signal is a separate reference signal that is derived from (but not equal to any of) the biomedical channel signals. In this case, the indicator could be a channel index of the reference channel with an indication that the reference channel is not one of biomedical channel signals, but an extra channel.

The exact approach or algorithm for deriving the reference signal from the biomedical channel signals will depend on the preferences and requirements of the individual embodiment.

In many embodiments, the determination circuit 103 may select one of the biomedical channel signals as the reference signal.

In other embodiments, the determination circuit 103 may be arranged to derive the reference signal from the biomedical channel signals, and specifically the reference signal may be generated as a combination of two or more, and indeed typically all, of the biomedical channel signals.

In many embodiments, the reference signal may be generated such that a variation and/or level/amplitude of the compensated signals is reduced, and preferably minimized. In particular, in many embodiments, the reference signal may be generated such that a combined entropy of the compensated biomedical channels signals is reduced, and preferably minimized.

In the context of signal compression and more generally information theory, entropy measures the amount of unpredictability or randomness in the data. Lower entropy means the data is more predictable and thus easier to encode and compress effectively.

In some embodiments, the determination circuit 103 may generate and evaluate a potentially large number of reference signals using different approaches and may for each signal generate compensated biomedical channel signals. It may then evaluate a variation/level/entropy measure for each compensated biomedical channel signal of a given set of biomedical channel signals that are to be coded relative to the reference signal. It may then generate a combined variation/level/entropy measure for the set of biomedical channel signals and proceed to select the reference signals as the signal that results in the lowest variation/level/entropy measure.

In some embodiments, the determination circuit (103) may, thus, generate the reference signal as a signal that reduces a difference measure which is indicative of a difference between the first reference signal and a set of the biomedical channel signals.

As another example, in some embodiments, the determination circuit 103 may comprise a trained neural network which as input receives the biomedical channel signals, or feature values that represent properties thereof, and may provide a reference signal as an output. Such a trained neural network may have been trained using a large sample of multichannel biomedical signals and using a range of different approaches for generating reference signals (e.g., including manual generation by human intervention) and using a loss function which is dependent on a variation/level/entropy measure generated for biomedical channel signals that have been compensated using the reference signal generated for the sample multichannel biomedical signal.

In many embodiments, the determination circuit 103 may be arranged to directly generate a reference signal from the multichannel biomedical signal without performing any compensation operation or evaluating any properties of resulting compensated biomedical channel signals. Rather, in some embodiments, the reference signal may be derived directly by applying a suitable function or algorithm to the biomedical channel signals of the multichannel biomedical signal.

In some embodiments, for example, the determination circuit 103 may be arranged to reduce the power/variance of differences between the biomedical channel signals and the reference signal. This may specifically be achieved by averaging the biomedical channel signals, and specifically the reference signal may be generated as the average signal value of the biomedical channel signals. Such a reference signal may also be referred to as the Common Average Reference (CAR).

In some embodiments where the reference signal is selected as one of the biomedical channel signals of the multichannel biomedical signal, the reference signal may for example be selected as the one for which the combined difference to the other biomedical channel signals (for which the reference signal is to be applied) is minimized. The biomedical channel signal may be selected as the one with the highest combined correlation to the other biomedical channel signals.

In some embodiments, the determination circuit 103 may directly determine the reference signal by selecting between a set of candidates, such as specifically the set of candidates consisting in the biomedical channel signals of the multichannel biomedical signal, based on a property of the signals.

For example, in some embodiments, the determination circuit 103 may determine the reference signal as the candidate signal for which a variation measure is minimized/the lowest. The variation measure may specifically be a variance, a standard deviation, or indeed an entropy measure.

In some embodiments, the determination circuit 103 may be based on a relative measure between the candidate biomedical channel signals. For example, in some embodiments, the determination circuit 103 may determine the reference signal as the biomedical channel signal having the highest combined correlation with the other biomedical channel signals.

The determination of the determination circuit 103 may be an automatic process based on factors such as signal type, number of channels, variance between channels, etc. The reference signal may be determined in different ways and in many cases different candidate options may be considered. For example, initially the CAR may be generated (as the sum/average of all biomedical channel signals). The reference signal may then e.g., be selected from the group comprising the CAR signal and the biomedical channel signals of the multichannel biomedical signal. The reference signal may for example be selected as the biomedical channel signals of:
- the channel with the highest correlation with all other channels,
- the channel with the lowest standard deviation,
- the channel with the lowest entropy,
- the channel which, after compensation, gives the lowest overall standard deviation across all channels, or
- the channel which, after compensation, gives the lowest overall entropy across all channels.

The evaluation of the variation/level/entropy may include the lossy or lossless compression stage.

In some embodiments, the encoder apparatus may as described proceed to include encoded reference signal data that describes the reference signal(s). In such cases, the decoder apparatus may directly decode the encoded reference signal data and then proceed to apply the generated reference signal to the received biomedical channel signals. It will be appreciated that in such cases, any suitable encoding and compression approach may be used for the reference signal. Typically, the same approach may be used for the reference signal and for each of the compensated biomedical channel signals. Indeed, in some cases, the reference signal may be a non-compensated biomedical channel signal of the multichannel biomedical signal.

In many cases, a single reference signal may be generated, and this may be used to compensate all (other) biomedical channel signals. However, in some embodiments, the compensation circuit 105 may be arranged to apply the compensation based on a given reference signal to only a subset of the biomedical channel signals. For example, the reference signal may only be applied to compensate biomedical channel signals that correspond to measurement electrodes close to an electrode used to generate the reference signal or may e.g., only be applied to biomedical channel signals that are highly correlated with the reference signal.

In some embodiments, a given reference signal may accordingly only be applied to a subset of biomedical channel signals and the multichannel biomedical signal may include data for other biomedical channel signals. In some such embodiments, these remaining biomedical channel signals may be included without any compensation. However, in many embodiments, the determination circuit 103 may be arranged to generate a plurality of reference signals and different biomedical channel signals may be encoded relative to different reference signals. Thus, a first subset of biomedical channel signals may be compensated using one reference signal and a second subset of biomedical channel signals, typically disjoint with the first subset of biomedical channel signals, may be compensated using a different reference signal.

In cases where multiple reference signals are generated, these may be derived from different subsets of biomedical channel signals. In particular, in many embodiments, the biomedical channel signals of the multichannel biomedical signal may be divided into different subsets and a reference signal may be generated for each individual subset. The reference signal may in such cases typically be used to compensate the biomedical channel signals for that subset. Thus, in many embodiments, the subset used for generating a reference signal and the subset for which the reference signal is used for compensation are identical. However, in some embodiments, the set of biomedical channel signals used for deriving a reference signal and the set of biomedical channel signals to which the reference signal is applied may be different.

As an example, in some embodiments, the received biomedical channel signals may be divided into a plurality of groups/subsets. The division may in some embodiments be based on position information and the subsets may e.g., be determined as groups of biomedical channel signals for measurement electrodes with a maximum distance between any two biomedical channel signals not exceeding a threshold. Alternatively, or additionally, the grouping into subsets may be based on the signal properties of the biomedical channel signals. For example, the cross-correlation between all possible pairs of biomedical channel signals may be determined and the biomedical channel signals may subsequently be grouped into e.g., a predetermined number of groups such that the lowest correlation between pairs of the same group is maximized. It will be appreciated that many algorithms are known and can be used for grouping, including more complex approaches such as e.g., using a clustering algorithm.

The determination circuit 103 may then proceed to derive a reference signal for each of the subsets, such as e.g., determining a CAR or using any of the previously described examples. The determined reference signal for each subset is then used to compensate the biomedical channel signals of that subset, such as typically by generating the compensated biomedical channel signals as difference signals relative to the reference signal. The resulting compensated biomedical channel signals may then be encoded as previously described.

Thus, in some embodiments, re-referencing may be applied to all biomedical channel signals of the multichannel biomedical signal, or may in some embodiments be applied to subsets of biomedical channel signals independently. In the latter case, there may be multiple reference signals with each of these being used to re-reference one or more subsets of the biomedical channel signals.

Thus, the re-referencing may happen on subsets of the biomedical channel signals, in which case for each subset, a reference signal can be derived e.g., as
- A CAR for the subset
- A biomedical channel signal of the subset with the lowest/highest standard deviation
- The biomedical channel signal of the subset with the lowest/highest entropy,
- The biomedical channel signal of the subset with the lowest/highest correlation
- A biomedical channel signal, or a signal derived therefrom, of the subset which after compensation results in compensated biomedical channel signals for the subset with a lowest variation measure, such as a lowest standard deviation, variance, or entropy.

The data signal generator 109 may be arranged to generate metadata that describes the subset operation. Specifically, the data signal generator 109 is arranged to include an indication of the division into subsets. In particular, it may provide an indication of the biomedical channel signals that are comprised in the individual subset. It may specifically provide such an indication for all subsets of the biomedical channel signals which have been differently compensated.

In particular, the data signal generator 109 may include an indication of each subset. It may further for each subset provide an indication of the reference signal and/or of a property of the algorithm that has been used to generate the reference signal. Specifically, the data signal generator 109 may proceed to include information corresponding to the compensation indication previously described, but with this information being specific to a given subset. This may be performed for each subset, and the data signal generator 109 may proceed to generate metadata that includes an indication of each subset and a compensation indication for each subset. The compensation indication may in many embodiments directly indicate the reference signal for the subset.

For example, the data signal generator 109 may proceed to generate metadata which for each subset first lists channel indices for all the biomedical channel signals in the subset followed by an indication of a channel index for a reference signal that is used to compensate that group.

In many embodiments, the reference signals for a plurality of subsets may be encoded and included in the biomedical data signal. The biomedical data signal may accordingly include compressed encoded data for a plurality of subsets. In such cases, the encoding of one reference signal may be relative to another reference signal. For example, a first reference signal may be fully encoded using a suitable encoding format as previously described. The encoder apparatus may further be arranged to decode this signal to generate a decoded version of the first reference signal. A second reference signal for a different group may then be compensated for the decoded first reference signal, and specifically the decoded first reference signal may be subtracted from the second reference signal. The resulting compensated/difference/relative signal may then be encoded using a suitable encoding algorithm and the resulting encoded data may be added to the biomedical data signal.

In such cases, the data signal generator 109 may further include metadata that includes an indication that the second reference signal is encoded relative to the first reference signal. The decoder apparatus may, when parsing the data signal, determine that two reference signals are present and may in response to the indication that the second reference signal is encoded relative to the first reference signal, proceed to first decode the first reference signal and the difference second reference signal. It may then generate the second reference signal by combining the first reference signal and the difference second reference signal, typically simply by adding these together.

In cases where the biomedical data signal includes three reference signals, this may be encoded relative to both the first and second reference signals. For example, the first reference signal and the difference second reference signal may be subtracted from the third reference signal and the resulting signal may be encoded. As another example, a linear combination of the first and second reference signals may be subtracted from the third reference signal and the remaining reference signal may be encoded. In such cases, weights of the linear combination may be determined to minimize the level/entropy of the compensated third reference signal. In such cases, the data signal generator 109 may generate the metadata to include an indication that the third reference signal is encoded relative to the first and second reference signals. In the example of the linear combination, the metadata may further include data indicating the weights of each of the first and second reference signals.

For some cases the re-referencing may happen in pairs or small clusters that are spatially located close to each other. This may be determined at the encoder, or be a pre-determined set of pairings/groupings. Referencing to neighboring channels has the benefit of removing noise from and spatially diffuse artifacts.

In some embodiments, the receiver 101 may further receive measurement position data for at least some of the biomedical channel signals. The measurement positions may typically be indications of a position on a body of the subject at which the electrode for the measurement is placed. The position information may be for relative positions with respect to positions for other channels. In some embodiments, the position information may be manually input. In some embodiments, measurement positions for the electrodes generating the biomedical channel signals may be predetermined. For example, for EEG or ECG, each electrode may be predetermined and marked for measurement at a particular position on the subject and the measurement is based on an assumption that the operator places the electrode at the correct position. In yet other embodiments, the encoder apparatus may be arranged to determine the position data based on the signal properties. For example, for an ECG application, and assuming the electrodes are positioned at predetermined nominal measurement positions, the biomedical channel signals may be compared to expected signals for these positions, and it can be determined which biomedical channel signal corresponds to which nominal measurement position.

In some embodiments, the determination circuit 103 is arranged to take the measurement positions into account when determining the reference signal(s).

For example, in some embodiments, the determination circuit 103 may determine the reference signal as the biomedical channel signal which is closest to the center of the measurement positions. In some embodiments, particular measurements may be suitable for providing a reference due to their particular position on the body, and the reference signal may be determined as the biomedical channel signal for the electrode closest to this position. In some embodiments, particularly measurement positions may be expected to essentially provide the same measurement result and the biomedical channel signals closest to such position may be selected and used to derive the reference signal (e.g., as the average thereof). In some embodiments, the reference signal may be determined as a linear combination of the surrounding electrodes of the biomedical channel signal(s).

In many cases, the position information may be used to group the biomedical channel signals into subsets. For example, as previously described, based on the position information, the biomedical channel signals may be grouped into subsets comprising biomedical channel signals for measurements that are in close proximity.

The apparatus(es) may specifically be implemented in one or more suitably programmed processors. The different functional blocks may be implemented in separate processors and/or may e.g., be implemented in the same processor. An example of a suitable processor is provided in the following.

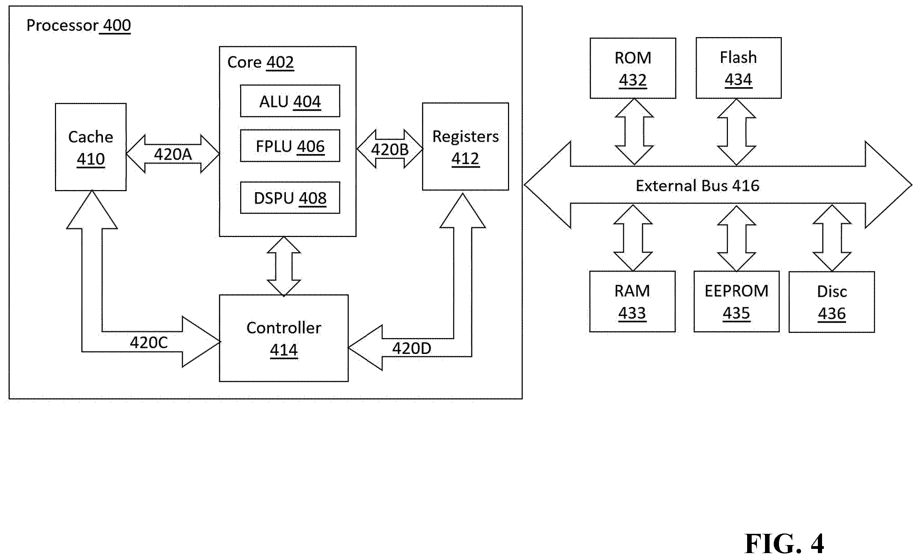

FIG. 4 is a block diagram illustrating an example processor 400 according to embodiments of the disclosure. Processor 400 may be used to implement one or more processors implementing an apparatus as previously described or elements thereof. Processor 400 may be any suitable processor type including, but not limited to, a microprocessor, a microcontroller, a Digital Signal Processor (DSP), a Field ProGrammable Array (FPGA) where the FPGA has been programmed to form a processor, a Graphical Processing Unit (GPU), an Application Specific Integrated Circuit (ASIC) where the ASIC has been designed to form a processor, or a combination thereof.

The processor 400 may include one or more cores 402. The core 402 may include one or more Arithmetic Logic Units (ALU) 404. In some embodiments, the core 402 may include a Floating Point Logic Unit (FPLU) 406 and/or a Digital Signal Processing Unit (DSPU) 408 in addition to or instead of the ALU 404.

The processor 400 may include one or more registers 412 communicatively coupled to the core 402. The registers 412 may be implemented using dedicated logic gate circuits (e.g., flip-flops) and/or any memory technology. In some embodiments the registers 412 may be implemented using static memory. The registers may provide data, instructions and addresses to the core 402.

In some embodiments, processor 400 may include one or more levels of cache memory 410 communicatively coupled to the core 402. The cache memory 410 may provide computer-readable instructions to the core 402 for execution. The cache memory 410 may provide data for processing by the core 402. In some embodiments, the computer-readable instructions may have been provided to the cache memory 410 by a local memory, for example, local memory attached to the external bus 416. The cache memory 410 may be implemented with any suitable cache memory type, for example, Metal-Oxide Semiconductor (MOS) memory such as Static Random Access Memory (SRAM), Dynamic Random Access Memory (DRAM), and/or any other suitable memory technology.

The processor 400 may include a controller 414, which may control input to the processor 400 from other processors and/or components included in a system and/or outputs from the processor 400 to other processors and/or components included in the system. Controller 414 may control the data paths in the ALU 404, FPLU 406 and/or DSPU 408. Controller 414 may be implemented as one or more state machines, data paths and/or dedicated control logic. The gates of controller 414 may be implemented as standalone gates, FPGA, ASIC or any other suitable technology.

The registers 412 and the cache 410 may communicate with controller 414 and core 402 via internal connections 420A, 420B, 420C and 420D. Internal connections may be implemented as a bus, multiplexer, crossbar switch, and/or any other suitable connection technology.

Inputs and outputs for the processor 400 may be provided via a bus 416, which may include one or more conductive lines. The bus 416 may be communicatively coupled to one or more components of processor 400, for example the controller 414, cache 410, and/or register 412. The bus 416 may be coupled to one or more components of the system.

The bus 416 may be coupled to one or more external memories. The external memories may include Read Only Memory (ROM) 432. ROM 432 may be a masked ROM, Electronically Programmable Read Only Memory (EPROM) or any other suitable technology. The external memory may include Random Access Memory (RAM) 433. RAM 433 may be a static RAM, battery backed up static RAM, Dynamic RAM (DRAM) or any other suitable technology. The external memory may include Electrically Erasable Programmable Read Only Memory (EEPROM) 435. The external memory may include Flash memory 434. The external memory may include a magnetic storage device such as disc 436. In some embodiments, the external memories may be included in a system.

The invention can be implemented in any suitable form including hardware, software, firmware or any combination of these. The invention may optionally be implemented at least partly as computer software running on one or more data processors and/or digital signal processors. The elements and components of an embodiment of the invention may be physically, functionally and logically implemented in any suitable way. Indeed, the functionality may be implemented in a single unit, in a plurality of units or as part of other functional units. As such, the invention may be implemented in a single unit or may be physically and functionally distributed between different units, circuits and processors.

Although the present invention has been described in connection with some embodiments, it is not intended to be limited to the specific form set forth herein. Rather, the scope of the present invention is limited only by the accompanying claims. Additionally, although a feature may appear to be described in connection with particular embodiments, one skilled in the art would recognize that various features of the described embodiments may be combined in accordance with the invention. In the claims, the term comprising does not exclude the presence of other elements or steps.

Furthermore, although individually listed, a plurality of means, elements, circuits or method steps may be implemented by e.g., a single circuit, unit or processor. Additionally, although individual features may be included in different claims, these may possibly be advantageously combined, and the inclusion in different claims does not imply that a combination of features is not feasible and/or advantageous. Also, the inclusion of a feature in one category of claims does not imply a limitation to this category but rather indicates that the feature is equally applicable to other claim categories as appropriate. Furthermore, the order of features in the claims does not imply any specific order in which the features must be worked, and in particular, the order of individual steps in a method claim does not imply that the steps must be performed in this order. Rather, the steps may be performed in any suitable order. In addition, singular references do not exclude a plurality. Thus, references to "a", "an", "first", "second" etc. do not preclude a plurality. Reference signs in the claims are provided merely as a clarifying example shall not be construed as limiting the scope of the claims in any way.

## Claims

1. An apparatus for generating a biomedical data signal, the apparatus comprising:
a receiver (101) arranged to receive a multichannel biomedical signal comprising a plurality of biomedical channel signals, each biomedical channel signal representing a measured differential voltage between two measurement electrodes;
a determination circuit (103) arranged to derive a first reference signal from the plurality of biomedical channel signals;
a compensation circuit (105) arranged to generate compensated biomedical channel signals by compensating at least some of the plurality of biomedical channels signals for the first reference signal;
a data encoder (107) arranged to generate encoded compressed data for the compensated biomedical channel signals; and
a data signal generator (109) arranged to generate the biomedical data signal to comprise the compressed encoded data.

2. The apparatus of any previous claim wherein the data signal generator (109) is arranged to include a compensation indication in the biomedical data signal, the compensation indication being indictive of a property of the compensation of the at least some of the plurality of biomedical channels signals.

3. The apparatus of any previous claim wherein the data encoder (107) is arranged to generate encoded reference signal data for the first reference signal; and the data signal generator (109) is arranged to generate the biomedical data signal to comprise the encoded reference signal data.

4. The apparatus of any previous claim wherein the determination circuit (103) is arranged to select a biomedical channel signal of the plurality of biomedical channel signals as the first reference signal.

5. The apparatus of any previous claim wherein the determination circuit (103) is arranged to generate the first reference signal as a combination of at least some of the biomedical channel signals.

6. The apparatus of any previous claim wherein the determination circuit (103) is arranged to generate the first reference signal as a signal that reduces a difference measure, the difference measure being indicative of a difference between the first reference signal and a set of signals from the plurality of biomedical channel signals.

7. The apparatus of any previous claim wherein the compensation circuit (105) is arranged to determine the at least some of the plurality of biomedical channels signals as a first subset of biomedical channel signals.

8. The apparatus of claim 7 wherein the data signal generator (109) is arranged to include an indication of the first subset in the biomedical data signal.

9. The apparatus of any previous claim 7 or 8 wherein the determination circuit (103) is arranged to derive a second reference signal from the plurality of biomedical channel signals; and the compensation circuit (105) is arranged to generate further compensated biomedical channel signals by compensating a second set of biomedical channel signals by the second reference signal; the data encoder (107) is arranged to generate further encoded compressed data for the further compensated biomedical channel signals; and the data signal generator (109) is arranged to generate the biomedical data signal to comprise the further compressed encoded data.

10. The apparatus of claim 9 wherein the data signal generator (109) is arranged to include an indication of the second subset in the biomedical data signal.

11. The apparatus of claim 8 or 10 wherein the data encoder (107) is arranged to generate further encoded compressed data by encoding the second reference signal relative to the first reference signal.

12. The apparatus of claim 11 wherein the data signal generator (109) is arranged to include an indication indicative of the second reference signal being encoded relative to the first reference signal.

13. The apparatus of any previous claim wherein the determination circuit (103) is arranged to determine the first reference signal in dependence on measurement positions for the measurement electrodes.

14. An apparatus for generating a multichannel biomedical signal, the apparatus comprising:
a receiver (201) arranged to receive a biomedical data signal comprising compressed encoded data for a multichannel biomedical signal comprising a plurality of biomedical channel signals, each biomedical channel signal representing a measured differential voltage between two measurement electrodes and the compressed encoded data representing an encoding of at least some of the biomedical channel signals relative to a reference signal;
a determination circuit (205) arranged to determine the reference signal from the biomedical data signal;
a decoding circuit (203) arranged to generate intermediate biomedical channel signals by decoding the compressed encoded data;
a circuit (207) arranged to generate the biomedical channel signals by compensating at least one of the intermediate biomedical channels by the reference signal; and
an output circuit (209) arranged to generate the multichannel biomedical signal to comprise the biomedical channel signals.

15. A method of generating a biomedical data signal, the method comprising:
receiving a multichannel biomedical signal comprising a plurality of biomedical channel signals, each biomedical channel signal representing a measured differential voltage between two measurement electrodes;
deriving a first reference signal from the plurality of biomedical channel signals;
generating compensated biomedical channel signals by compensating at least some of the plurality of biomedical channels signals for the first reference signal;
generating encoded compressed data for the compensated biomedical channel signals;
generating the biomedical data signal to comprise the compressed encoded data.

16. A method of generating a multichannel biomedical signal, the method comprising:
receiving a biomedical data signal comprising compressed encoded data for a multichannel biomedical signal comprising a plurality of biomedical channel signals, each biomedical channel signal representing a measured differential voltage between two measurement electrodes and the compressed encoded data representing an encoding of at least some of the biomedical channel signals relative to a reference signal;
determining the reference signal from the biomedical data signal;
generating intermediate biomedical channel signals by decoding the compressed encoded data;
generating the biomedical channel signals by compensating at least one of the intermediate biomedical channels by the reference signal; and
generating the multichannel biomedical signal to comprise the biomedical channel signals.

17. A computer program product comprising computer program code means adapted to perform all the steps of claims 15 or 16 when said program is run on a computer.
